(19) Europäisches Patentamt
European Patent Office
Office européen des brevets

(11) Publication number: 0 185 554 B1

# (12) EUROPEAN PATENT SPECIFICATION

(45) Date of publication of the patent specification: 01.03.89

(51) Int. Cl.⁴: C 07 C 43/12, C 07 C 43/225, C 07 C 41/22, C 07 C 41/03

(21) Application number: 85309288.0

(22) Date of filing: 19.12.85

## (54) Preparation of alkoxy halides.

(30) Priority: 20.12.84 GB 8432277

(43) Date of publication of application: 25.06.86 Bulletin 86/26

(45) Publication of the grant of the patent: 01.03.89 Bulletin 89/9

(84) Designated Contracting States: BE CH DE FR GB IT LI NL

(56) References cited:
EP-A- 0 085 167
CH-A- 385 386
FR-A- 1 381 786

JOURNAL OF ORGANIC CHEMISTRY, vol. 40, no. 1, 10th January 1975, pages 134-136; T.G. SQUIRES et al.: "Zinc chloride catalysis in the reaction of thionyl halides with aliphatic alcohols"

(73) Proprietor: The British Petroleum Company p.l.c., Britannic House Moor Lane, London EC2Y 9BU (GB)

(72) Inventor: Hodgson, Philip Kenneth Gordon, The British Petroleum Co. p.l.c. Chertsey Road, Sunbury-on-Thames Middlesex, TW16 7LN (GB)
Inventor: Stweart, Nevin John, The British Petroleum Co. p.l.c. Chertsey Road, Sunbury-on-Thames Middlesex, TW16 7LN (GB)

(74) Representative: MacLeod, Malcolm et al, BP INTERNATIONAL LIMITED Patents Division Chertsey Road, Sunbury-on-Thames Middlesex TW16 7LN (GB)

EP 0 185 554 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for the preparation of alkyl alkoxy, aryl alkoxy or alkylaryl alkoxy halides which are suitable for use as intermediates in the preparation of surfactants.

Numerous attempts have been made to develop surfactant compositions for use in enhanced oil recovery and the patent literature is replete with descriptions of formulations, see for example, USP's 4 424 135, 4 159 037, 4 110 228, 4 066 124 and 4 018 278.

A useful summary of the art is given in Kirk-Othmer's Encyclopedia of Chemical Technology, Third Edition, Volume 17, pages 168–182. This indicates that most compositions contain (a) a main surfactant which is either a petroleum sulphonate or a synthetic hydrocarbyl sulphonate and (b) co-surfactants which include simple alcohols, ethoxylated alcohols and sulphated ethoxylated alcohols.

It has also been disclosed that alkyl and alkylaryl polyalkoxy alkylene sulphonates may be used as co-surfactants. These compounds are generally prepared by a three-stage process. In the first stage of a typical process an alcohol or alkyl phenol is condensed with an alkylene oxide in the presence of sodium or potassium hydroxide to form an alkoxylate. This is then halogenated by treatment with thionyl or sulphuryl chloride, usually in the absence of a catalyst. Finally the halide is converted to a sulphonate by reaction with sodium sulphite, again, usually in the absence of a catalyst.

The alkali catalyst must be removed after the alkoxylation reaction since its presence is harmful to the stability of the product.

We have now discovered a process for the production of alkoxy halides from alcohols or phenols in which a single soluble inorganic catalyst is employed.

Thus according to the present invention there is provided a process for the production of an alkyl alkoxy, aryl alkoxy or an alkylaryl alkoxy halide which process comprises reacting an alcohol of formula $R^1$–OH, phenol or an alkyl phenol of formula:

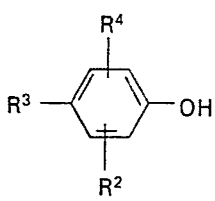

wherein

$R^1$ is an alkyl group containing 1 to 24, preferably 8 to 20, carbon atoms,

$R^2$ is an alkyl group containing 1 to 24, preferably 8 to 20, carbon atoms and

$R^3$ and $R^4$ are hydrogen atoms, or

$R^2$ and $R^3$ are both alkyl groups containing 1 to 12 carbon atoms and

$R^4$ is a hydrogen atom, or

$R^2$, $R^3$ and $R^4$ are each alkyl groups wherein the sum of the number of carbon atoms in the groups is in the range 3 to 24, with ethylene oxide and/or proplyene oxide in the presence of a soluble or solubilised compound of a metal of Groups IA or IIA of the Periodic Table as a catalyst, to form an alkyl alkoxy, aryl alkoxy or alkylaryl alkoxy alcohol, containing 1 to 15, preferably 4 to 10, alkoxy units per molecule, and directly reacting the reaction product with a halogenating agent in the continuing presence of the catalyst.

Suitable catalysts include the chlorides, hydroxides, alkoxides and alkanoates of lithium, magnesium, calcium, strontium and barium.

Other suitable catalysts include the above compounds of sodium, potassium, rubidium and caesium solubilised by a solubilising agent. Suitable agents include dipolar aprotic solvents such as dimethyl sulphoxide, crown ethers and polyalkoxy compounds containing three or more alkoxy groups per molecule such as dimethyl polyethylene glycol.

A catalyst promoter may be employed if desired. Known promoters for alkoxylation catalysts include phenols, carboxylic acids, polyols, aldehydes, ketones, amines, amides and inorganic acids.

The alkoxy distribution may be determined by the choice of catalyst.

If a broad distribution is desired then the catalyst is preferably a lithium compound.

If, on the other hand, a narrow distribution is preferred then the catalyst is preferably a compound of a Group IIA metal together with a promoter.

Preferred halogenating agents are chlorinating agents such as thionyl chloride and sulphuryl chloride.

A pressure in the range of atmospheric to 7 bar may be employed for the alkoxylation stage.

Pressure is not a significant parameter. for the halogenation stage and this reaction is therefore most conveniently carried out at atmospheric pressure.

Alkoxylation is suitably carried out at a temperature in the range 100 °to 260 °C, preferably 120 ° to 190 °C, and halogenation is suitably effected at a temperature in the range 30° to 120 °C, preferably 75° to 85 °C.

The molar ratio of alkoxy alcohol to halogenating agent in the halogenation stage is suitably in the range 1:1 to 1:5, preferably 1:1 to 1:1.5.

The quantity of catalyst employed is preferably in the range 0.1 to 10% by weight, preferably 0.2 to 1%, expressed as a percentage by weight of the alcohol, phenol or alkyl phenol.

The reactions may be effected in the presence or, preferably, the absence of a solvent. Suitable solvents include 1,2-dichloroethane, toluene and chloroform.

The process according to the present invention possesses the following advantages:

(1) A single catalyst can be used for both alkoxylation and halogenation reactions thus saving

the cost of employing separate catalyst for each reaction;

(2) Neutralisation of the product and/or catalyst removal from the alkoxylation stage is no longer necessary.

(3) Hydrated salts or bases do not require special drying (water is usually removed after catalyst addition in alkoxylation reactions prior to the introduction of the alkylene oxide).

(4) Salts or bases are slow to dissolve in the halogenation mixture if added at this stage and thus the halogenation reaction may be adversely affected. When, however, the catalyst is previously employed in the alkoxylation stage, it is already solubilised.

(5) Cheaper compounds such as hydroxides or alkoxides may be employed as catalysts.

(6) The catalyst is readily recoverable by filtration or by extraction with water.

As a result of the reduced reaction time in the halogenation stage, the tendency of the polyoxyalkylene chain to cleave, liberating 1,4-dioxan in the case of a polyoxyethylene chain as noted by Robinson et al, J. Soc. Cosmet. Chem., 31, 329–337, is reduced. Thus both the yield and selectivity as indicated by the matching of the alkoxy distribution in the alcohol and the halide are improved and product contamination with unwanted by-product is reduced.

The invention is illustrated with reference to the following Examples. Example 2 is not in accordance with the invention and is provided for comparison.

Example 1

Hexadecanol was treated with strontium hydroxide (1% w/w) and phenol (1.5% w/w) at 100 °C for one hour under vacuum to remove water to a level of 0.07% w/w. It was then reacted with ethylene oxide at 125 °C under 4 bar pressure for 5 hours to incorporate 4 moles ethylene oxide per mole alcohol.

The unrefined ethoxyalcohol $C_{16}H_{33}(OCH_2CH_2)_4OH$ (54.8 g, 0.131M) was heated with stirring at 80 °C with thionyl chloride (16.8 g, 0.141M).

$^{13}C$ NMR (Nuclear Magnetic Resonance) spectroscopy indicated 90% conversion to ethoxychloride after 2 hours and GLC (Gas Liquid Chromatography) indicated the production of 0.02 moles of 1,4-dioxan per mole ethoxyalcohol.

Example 2

Hexadecanol was treated with potassium hydroxide (0.3% w/w) at 100 °C, under vacuum to remove water. It was then reacted with ethylene oxide at 125 °C under 4 bar pressure for 5 hours to incorporate 4 moles ethylene oxide per mole alcohol.

The unrefined ethoxyalcohol $C_{16}H_{33}(OCH_2CH_2)_4OH$ (50.0 g, 0.120M) was heated with stirring at 80 °C with thionyl chloride (15.4 g, 0.129M).

$^{13}C$ NMR spectroscopy indicated 26% conversion to ethoxychloride after 2 hours and GLC indicated the production of 0.2 moles of 1,4-dioxan per mole ethoxyalcohol.

It should be noted when comparing Examples 1 and 2 that the effective ethoxylation-chlorination catalyst of Example 1, (strontium ion), resulted in a chlorination rate 3.5 times greater than in Example 2, the case where the ethoxylation catalyst is an ineffective chlorination catalyst. 1,4-dioxan production was 10 times greater in the uncatalysed chlorination than in the catalysed chlorination.

A low chlorination rate, similar to that of Example 2, would be expected if the potassium compound were removed following ethoxylation. This is usually performed as a routine operation.

The examples illustrate the value of leaving suitable ethoxylation catalysts in the ethoxyalcohol to effect catalysis of the chlorination stage.

## Claims

1. A process for the production of an alkyl alkoxy, aryl alkoxy or an alkylaryl alkoxy halide which process comprises reacting an alcohol of formula $R^1$–OH, phenol or an alkyl phenol of formula:

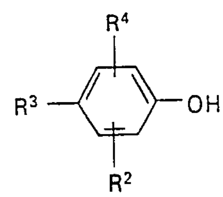

wherein

$R^1$ is an alkyl group containing 1 to 24 carbon atoms,

$R^2$ is an alkyl group containing 1 to 24 carbon atoms and

$R^3$ and $R^4$ are hydrogen atoms, or

$R^2$ and $R^3$ are both alkyl groups containing 1 to 12 carbon atoms and

$R^4$ is a hydrogen atom, or

$R^2$, $R^3$ and $R^4$ are each alkyl groups wherein the sum of the number of carbon atoms in the groups is in the range 3 to 24, with ethylene oxide and/or propylene oxide to form an alkyl alkoxy, aryl alkoxy or alkylaryl alkoxy alcohol, containing 1 to 15 alkoxy units per molecule, characterised by the fact that the reaction is carried out in the presence of a soluble or solubilised compound of a metal of Groups IA or IIA of the Periodic Table as a catalyst, and the reaction product is directly reacted with a halogenating agent in the continuing presence of the catalyst.

2. A process according to claim 1 wherein $R^1$ is an alkyl group containing 8 to 20 carbon atoms, or $R^2$ is an alkyl group containing 8 to 20 carbon atoms and $R^3$ and $R^4$ are hydrogen atoms, and the alkoxy moiety contains 4 to 10 alkoxy units per molecule.

3. A process according to either of the preceding claims wherein the catalyst is selected from the group consisting of the chlorides, hydroxides, alkoxides and alkanoates of lithium, magnesium, calcium, strontium and barium.

4. A process according to either of claims 1 or 2 wherein the catalyst is selected from the group consisting of the chlorides, hydroxides, alkoxides and alkanoates of sodium, potassium, rubidium and caesium solubilised by a solubilising agent.

5. A process according to claim 4 wherein the solubilising agent is selected from the group consisting of dipolar aprotic solvents, crown ethers and polyalkoxy compounds containing three or more alkoxy groups per molecule.

6. A process according to any of the preceding claims wherein the alkoxylation reaction is effected at a temperature in the range 100° to 260 °C.

7. A process according to any of the preceding claims wherein the alkoxylation reaction is effected at a pressure in the range atmospheric to 7 bar.

8. A process according to any of the preceding claims wherein the halogenation reaction is effected at a temperature in the range 30° to 120 °C.

9. A process according to any of the preceding claims wherein the molar ratio of alkoxy alcohol to halogenating agent is in the range 1:1 to 1:5.

10. A process according to any of the preceding claims wherein the halogenating agent is thionyl chloride or sulphuryl chloride.

## Patentansprüche

1. Verfahren zur Herstellung eines Alkylalkoxy-, Arylalkoxy- oder Alkylarylalkoxy-Halogenids, umfassend die Umsetzung eines Alkohols der Formel $R^1$–OH, eines Phenols oder eines Alkylphenols der Formel

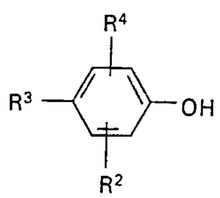

in der

$R^1$ eine Alkyl-Gruppe mit 1 bis 24 Kohlenstoff-Atomen ist,

$R^2$ eine Alkyl-Gruppe mit 1 bis 24 Kohlenstoff-Atomen ist und

$R^3$ und $R^4$ Wasserstoff-Atome sind oder

$R^2$ und $R^3$ beide Alkyl-Gruppen mit 1 bis 12 Kohlenstoff-Atomen sind und

$R^4$ ein Wasserstoff-Atom ist oder

$R^2$, $R^3$ und $R^4$ jeweils Alkyl-Gruppen sind, wobei die Summe der Zahl der Kohlenstoff-Atome in den Gruppen im Bereich von 3 bis 24 liegt, mit Ethylenoxid und/oder Propylenoxid unter Bildung eines Alkylalkoxy-, Arylalkoxy- oder Alkylarylalkoxyalkohols mit 1 bis 15 Alkoxy-Einheiten im Molekül, dadurch gekennzeichnet, dass die Reaktion in Gegenwart einer löslichen oder solubilisierten Verbindung eines Metalls der Gruppen IA oder IIA des Periodensystems als Katalysator durchgeführt wird und das Reaktionsprodukt unmittelbar mit einem Halogenierungsmittel bei fortdauernder Anwesenheit des Katalysators umgesetzt wird.

2. Verfahren nach Anspruch 1, worin

$R^1$ eine Alkyl-Gruppe mit 8 bis 20 Kohlenstoff-Atomen ist oder

$R^2$ eine Alkyl-Gruppe mit 8 bis 20 Kohlenstoff-Atomen ist und

$R^3$ und $R^4$ Wasserstoff-Atome sind und die Alkoxy-Struktureinheit 4 bis 10 Alkoxy-Einheiten im Molekül enthält.

3. Verfahren nach einem der vorhergehenden Ansprüche, worin der Katalysator aus der aus den Chloriden, Hydroxiden, Alkoxiden und Alkanoaten von Lithium, Magnesium, Calcium, Strontium und Barium bestehenden Gruppe ausgewählt ist.

4. Verfahren nach einem der Ansprüche 1 oder 2, worin der Katalysator aus der aus den Chloriden, Hydroxiden, Alkoxiden und Alkanoaten von Natrium, Kalium, Rubidium und Caesium, die mittels eines Lösungsvermittlers solubilisiert wurden, bestehenden Gruppe ausgewählt ist.

5. Verfahren nach Anspruch 4, worin der Lösungsvermittler aus der aus dipolaren aprotischen Lösungsmitteln, Kronenethern und Polyalkoxy-Verbindungen mit drei oder mehr Alkoxy-Gruppen im Molekül bestehenden Gruppe ausgewählt ist.

6. Verfahren nach irgendeinem der vorhergehenden Ansprüche, worin die Alkoxylierungsreaktion bei einer Temperatur im Bereich von 100 °C bis 260 °C durchgeführt wird.

7. Verfahren nach irgendeinem der vorhergehenden Ansprüche, worin die Alkoxylierungsreaktion bei einem Druck im Bereich von Atmosphärendruck bis 7 bar durchgeführt wird.

8. Verfahren nach irgendeinem der vorhergehenden Ansprüche, worin die Halogenierungsreaktion bei einer Temperatur im Bereich von 30 °C bis 120 °C durchgeführt wird.

9. Verfahren nach irgendeinem der vorhergehenden Ansprüche, worin das Stoffmengen-Verhältnis («Mol-Verhältnis») Alkoxyalkohol zu Halogenierungsmittel im Bereich von 1:1 bis 1:5 liegt.

10. Verfahren nach irgendeinem der vorhergehenden Ansprüche, worin das Halogenierungsmittel Thionylchlorid oder Sulfurylchlorid ist.

## Revendications

1. Procédé pour la production d'un halogénure d'alcoxy alkyle, d'un halogénure d'alcoxy aryle ou d'un halogénure d'alcoxy alkylaryle, ce procédé comprenant la réaction d'un alcool de formule $R^1$–OH, d'un phénol ou d'un alkylphénol de formule:

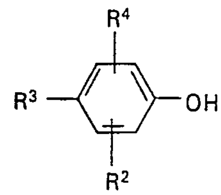

dans laquelle

$R^1$ représente un groupe alkyle contenant 1 à 24 atomes de carbone,

$R^2$ représente un groupe alkyle contenant 1 à 24 atomes de carbone, et

$R^3$ et $R^4$ représentent des atomes d'hydrogène, ou bien

$R^2$ et $R^3$ représentent tous deux des groupes alkyles contenant 1 à 12 atomes de carbone et $R^4$ représente un atome d'hydrogène ou bien

$R^2$, $R^3$ et $R^4$ représentent chacun des groupes alkyles, la somme du nombre des atomes de carbone présents dans les groupes se situant entre 3 et 24, avec l'oxyde d'éthylène et/ou l'oxyde de propylène pour former un alkyl alcoxy alcool, un aryl alcoxy alcool ou un alkylaryl alcoxy alcool, contenant 1 à 15 motifs alcoxy par molécule, procédé caractérisé par le fait que l'on conduit la réaction en présence d'un composé, soluble ou solubilisé, d'un métal des groupes IA ou IIA du Tableau Périodique, à titre de catalyseur, et en ce qu'on fait directement réagir le produit de cette réaction avec un agent d'halogénation, en présence constante du catalyseur.

2. Procédé selon la revendication 1, dans lequel $R^1$ représente un groupe alkyle contenant 8 à 20 atomes de carbone, ou bien $R^2$ représente un groupe alkyle contenant 8 à 20 atomes de carbone et $R^3$ et $R^4$ représentent des atomes d'hydrogène, et le fragment alcoxy contient 4 à 10 motifs alcoxy par molécule.

3. Procédé selon l'une ou l'autre des revendications précédentes, dans lequel le catalyseur est choisi dans l'ensemble constitué par les chlorures, hydroxydes, alcoolates et alcanoates de lithium, de magnésium, de calcium, de strontium et de baryum.

4. Procédé selon l'une ou l'autre des revendications 1 ou 2, dans lequel le catalyseur est choisi dans l'ensemble constitué par les chlorures, les hydroxydes, alcoolates et alcanoates de sodium, de potassium, de rubidium et de césium, solubilisés par un agent de solubilisation.

5. Procédé selon la revendication 4, dans lequel l'agent de solubilisation est choisi dans l'ensemble constitué par des solvants aprotiques dipolaires, des éthers-couronnes et des composés polyalcoxylés contenant trois ou plus de trois groupes alcoxy par molécule.

6. Procédé selon l'une quelconque des revendications précédentes, dans lequel on effectue la réaction d'alcoxylation à une température comprise entre 100 °C et 260 °C.

7. Procédé selon l'une quelconque des revendications précédentes, dans lequel on effectue la réaction d'alcoxylation à une pression comprise entre la pression atmosphérique et 7 bars.

8. Procédé selon l'une quelconque des revendications précédentes, dans lequel on effectue la réaction d'halogénation à une température comprise entre 30 et 120 °C.

9. Procédé selon l'une quelconque des revendications précédentes, dans lequel le rapport molaire de l'alcoxy alcool à l'agent d'halogénation se situe entre 1:1 et 1:5.

10. Procédé selon l'une quelconque des revendications précédentes, dans lequel l'agent d'halogénation est le chlorure de thionyle ou le chlorure de sulfuryle.